# EUROPEAN PATENT APPLICATION

(11) **EP 4 830 675 A1**
(43) Date of publication of application: **28.10.2026**
(21) Application number: 25861202.7
(22) Date of filing: 05.08.2025
(51) Int. Cl.: A61F 5/44, A61F 5/451, G01K 7/00, G01K 13/02, H04W 4/70

(54) **SYSTEM FOR COLLECTING EXCREMENT HAVING TEMPERATURE MONITORING FUNCTION**

(30) Priority: 02.09.2024 KR 20240118748
(71) Applicant: Woochang Medical Co., Ltd, Gimpo-si, Gyeonggi-do 10039 (KR)
(72) Inventor: JEON, Du Han, Gimpo-si, Gyeonggi-do 10064 (KR)
(74) Representative: BCKIP Part mbB
(86) International application number: PCT/KR2025/011660
(87) International publication number: WO 2026/049338

(57) **Abstract**

The present disclosure relates to an excrement collection system having a temperature monitoring function, which includes a mounting unit to be adhered to skin, a receptacle configured to accommodate excrement, and a temperature measurement unit including a signal line, a thermistor, and a contact pad, thereby enabling efficient monitoring of a temperature of excrement, reducing manufacturing costs, and improving convenience for a wearer.

## Description

### Technical Field

An embodiment of the present disclosure relates to an excrement collection system having a temperature monitoring function.

### Background Art

Excrement collection apparatuses for collecting urine, feces, and the like are known as products designed to be primarily worn by incontinent patients and particularly, bedridden patients.

Such excrement collection apparatuses are configured to be attached to an anal or genital region of a wearer to collect and immediately receive urine, feces, and other bodily excretions. Patent Document 1 and the like disclose a technology for monitoring the temperature of feces and the like while collecting the feces and the like.

However, although conventional excrement disposal apparatuses are capable of monitoring the temperature of excrement, there are limitations in minimizing the discomfort of a wearer, and there are difficulties in efficiently mass-producing an excrement collection apparatus including components for monitoring the temperature of excrement and in reducing manufacturing costs.

The foregoing background art is technical information that the inventor possessed for deriving the present disclosure or acquired in the course of deriving the present disclosure, and does not necessarily constitute prior art disclosed to the general public prior to the filing of the present disclosure.

Patent Document 1: KR10-2581447B

### Disclosure of Invention

### Technical Problem

An aspect of the present disclosure provides an excrement collection system having a temperature monitoring function, which is capable of efficiently monitoring the temperature of excrement while reducing manufacturing costs and improving wearer convenience.

### Solution to Problem

An excrement collection system having a temperature monitoring function according to an exemplary embodiment of the present disclosure may include: a mounting unit having one surface to be adhered to a user's skin and a through-portion provided at an inner side thereof; an excrement receptacle coupled to another surface of the mounting unit and configured to accommodate excrement; and a temperature measurement unit configured to measure a temperature of the excrement. The temperature measurement unit may include: a signal line including a first part and a second part extending from another end of the first part; a thermistor provided at one end of the first part; and a contact pad provided at another end of the second part, wherein the first part may be positioned inside the through-portion, and at least a portion of the second part may be coupled to the mounting unit.

In addition, a length obtained by subtracting a radius of the through-portion from a length of the first part may be 10 mm to 20 mm.

In addition, the mounting unit may include a main sheet part having a film layer coupled to another surface thereof.

In addition, the temperature measurement unit may include a cladding layer provided outside the signal line to ensure insulation, and an auxiliary film covering at least a portion of the cladding layer. One surface of the excrement receptacle may be coupled to the mounting unit while in contact with the film layer, the cladding layer, and the auxiliary film, and the excrement receptacle, the film layer, and the auxiliary film may be formed of the same material.

In addition, a concave portion may be provided at a 12 o'clock position of the mounting unit, the contact pad may be positioned at a 10 to 11 o'clock position or a 1 to 2 o'clock position of the mounting unit, convex portions may be provided at 3 o'clock and 9 o'clock positions of the mounting unit, and a connection portion may be provided at a 6 o'clock position of the mounting unit.

In addition, the excrement collection system may further include: a first terminal device removably provided on the contact pad and configured to generate temperature-related information by processing a current of the signal line; and a second terminal device connected to the first terminal device via a wired or wireless communication network to collect the temperature-related information.

### Advantageous Effects of Invention

According to an embodiment of the present disclosure, by utilizing a thermistor included in a temperature measurement unit, it is possible to accurately collect, in real-time, a temperature inside an anus or a temperature change occurring as excrement contacts the thermistor outside the patient's body.

In addition, temperature monitoring results can be collected by connecting a terminal device to a contact pad electrically connected to the thermistor. Accordingly, excrement can be collected while temperature monitoring is performed by discarding a used excrement collector and connecting a contact pad of a new excrement collector to the terminal device, thereby reducing costs associated with single-use operation of the excrement collector.

Furthermore, discomfort of a wearer may be reduced by optimizing the position and orientation of the temperature measurement unit.

### Brief Description of Drawings

FIG. 1 is a schematic perspective view illustrating an excrement collector according to an embodiment of the present disclosure,
FIG. 2 is a schematic exploded perspective view of FIG. 1,
FIG. 3 is a diagram for explaining an excrement receptacle,
FIG. 4 is a diagram for explaining an excrement collection system having a temperature monitoring function, according to an embodiment of the present disclosure,
FIG. 5 is a diagram for explaining a cross-sectional structure of an excrement collection system having a temperature monitoring function, according to an embodiment of the present disclosure,
FIG. 6 is a diagram schematically illustrating a cross-section taken along line A-A' of FIG. 5,
FIG. 7 is a diagram for explaining a temperature measurement unit module,
FIG. 8 is a diagram for explaining a temperature measurement unit,
FIG. 9 is a diagram for explaining an excrement collection system having a temperature monitoring function, according to an embodiment of the present disclosure,
FIG. 10 is a diagram for explaining an excrement collection system having a temperature monitoring function, according to another embodiment of the present disclosure, and
FIG. 11 is a diagram for explaining a modified example of an excrement collection system having a temperature monitoring function.

### Mode for the Invention

Advantages and features of the present disclosure and a method for achieving them will be apparent with reference to embodiments described below together with the accompanying drawings. However, the present disclosure is not limited to the embodiments disclosed below, but may be implemented in various forms. These embodiments are provided such that the present disclosure will be thorough and complete, and will fully convey the concept of the present disclosure to those of skill in the art. Like reference numerals denote like elements throughout the specification.

Terms used herein are for the purpose of describing embodiments, and are not intended to limit the present disclosure. The singular expression used herein also includes the plural meaning unless mentioned otherwise. The terms 'comprise' and/or 'comprising' as used herein do not preclude the presence or addition of one or more other components, steps, operations, and/or elements.

Although the terms 'first,' 'second,' and the like are used to describe different elements or components, these elements or components are not limited by these terms. These terms are only used to distinguish one element or component from another. Therefore, a first element or component mentioned below could be termed a second element or component without departing from the technical spirit of the present disclosure.

Unless otherwise defined, all terms (including technical and scientific terms) used herein have the same meaning as commonly understood by one of ordinary skill in the art to which the present disclosure pertains. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, the configuration and effects of the present disclosure will be described in more detail with reference to the accompanying drawings.

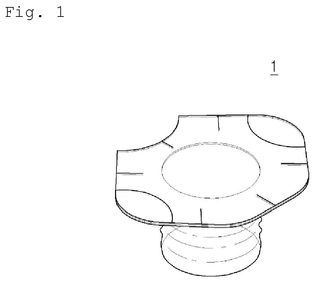

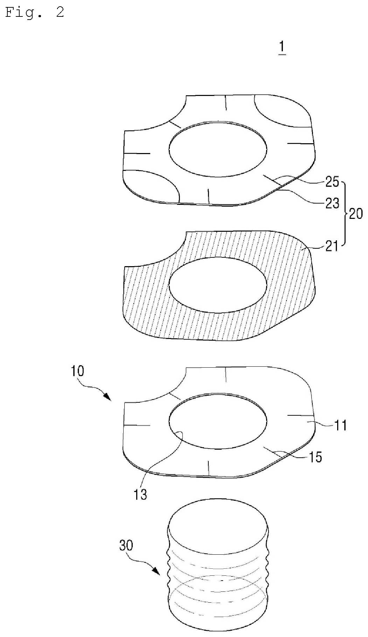

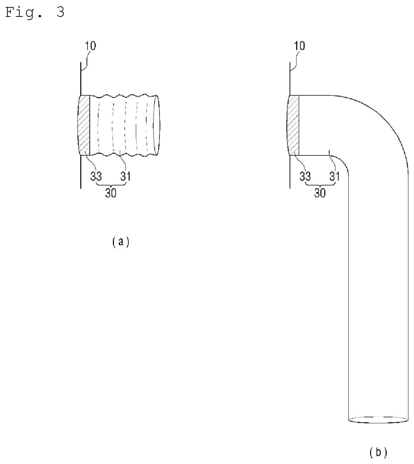

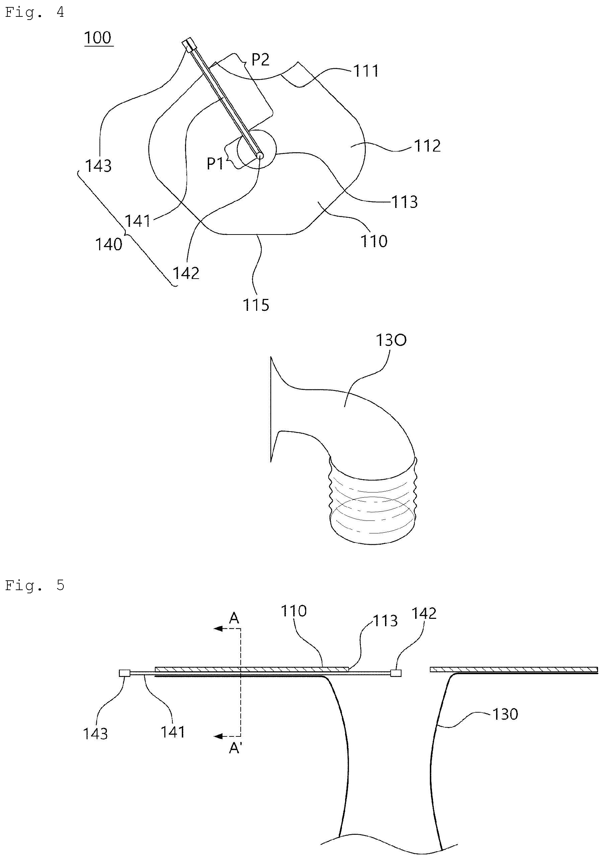

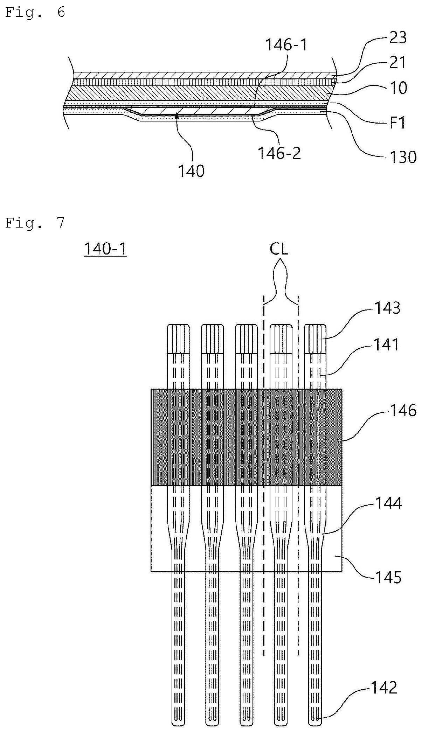

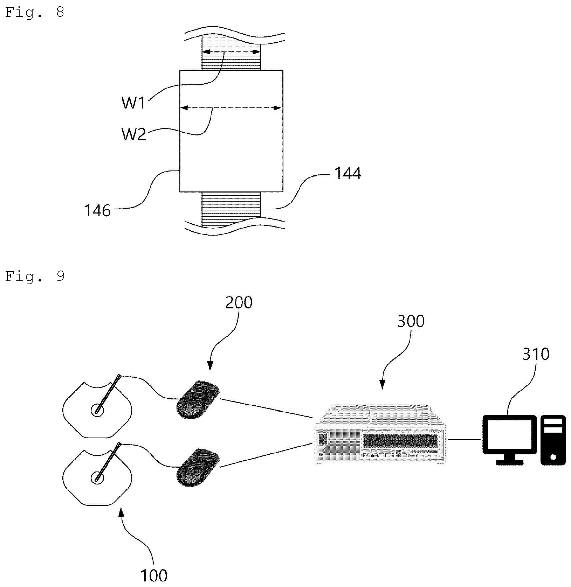

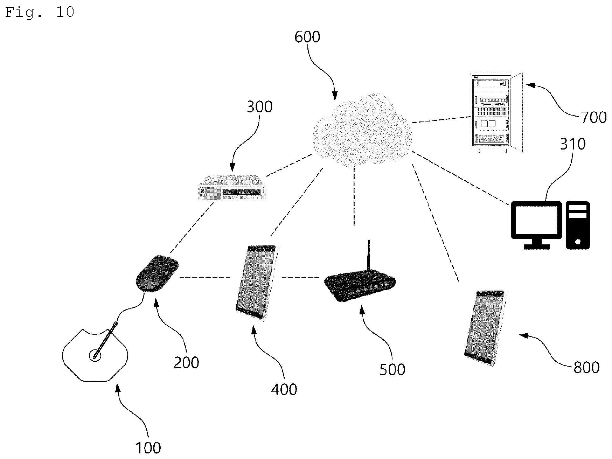

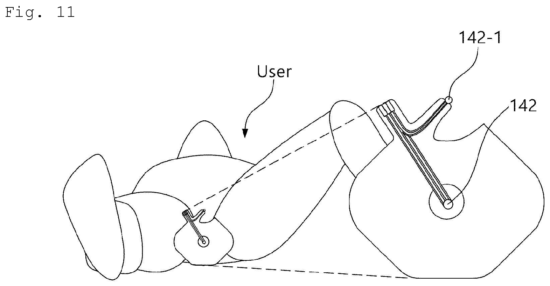

FIG. 1 is a schematic perspective view illustrating an excrement collector 1 according to an embodiment of the present disclosure, FIG. 2 is a schematic exploded perspective view of FIG. 1, FIG. 3 is a diagram for explaining an excrement receptacle 30, FIG. 4 is a diagram for explaining an excrement collection system having a temperature monitoring function, according to an embodiment of the present disclosure, FIG. 5 is a diagram for explaining a cross-sectional structure of an excrement collection system having a temperature monitoring function, according to an embodiment of the present disclosure, FIG. 6 is a diagram schematically illustrating a cross-section taken along line A-A' of FIG. 5, FIG. 7 is a diagram for explaining a temperature measurement unit module 140-1, FIG. 8 is a diagram for explaining a temperature measurement unit 140, FIG. 9 is a diagram for explaining an excrement collection system having a temperature monitoring function, according to an embodiment of the present disclosure, FIG. 10 is a diagram for explaining an excrement collection system having a temperature monitoring function, according to another embodiment of the present disclosure, and FIG. 11 is a diagram for explaining a modified example of an excrement collection system having a temperature monitoring function.

Hereinafter, embodiments will be described in detail with reference to the accompanying drawings. However, because various modifications may be made to the embodiments, the scope of the present disclosure is not limited or restricted by these embodiments. It should be understood that all modifications, equivalents, and substitutes for the embodiments are included in the scope of the present disclosure.

Specific structural or functional descriptions of the embodiments are disclosed for illustrative purposes only and may be implemented in various modified forms. Therefore, the embodiments are not limited to specific modes of practice, and the scope of the present specification includes modifications, equivalents, or substitutes falling within the technical spirit.

While the terms "first," "second," etc. may be used to describe various elements, these terms should be construed only for the purpose of distinguishing one element from another. For example, a first element may be referred to as a second element, and similarly, a second element may be referred to as a first element.

It is to be understood that when a certain element is referred to as being "connected to" another element, the element may be connected to the other element directly, but may also be understood as having a different element therebetween.

The terminology used in the embodiments is for the purpose of describing particular embodiments only and is not intended to be limiting. Singular expressions include plural expressions unless the context clearly indicates otherwise. In the present specification, it is to be understood that the terms such as "comprise," "include," or "have" are used to designate a presence of characteristic, number, stage, operation, element, component, or a combination thereof, and not to preclude a presence or a possibility of adding one or more of other characteristics, numbers, stages, operations, elements, components or a combination thereof.

Unless otherwise defined, all terms used herein, including technical and scientific terms, have the same meaning as commonly understood by those of skill in the art to which the embodiments pertain based on an understanding of the disclosure. Terms, such as those defined in commonly used dictionaries, are to be interpreted as having a meaning that is consistent with their meaning in the context of the relevant art and the disclosure, and are not to be interpreted in an idealized or overly formal sense unless expressly so defined herein.

In the description with reference to the accompanying drawings, the same elements are assigned the same reference numerals irrespective of the drawing numbers, and redundant descriptions thereof will be omitted. In the description of an embodiment, a detailed description of relevant well-known techniques will be omitted when it unnecessarily obscures the gist of the embodiment.

Advantages and features of the present disclosure and a method for achieving them will be apparent with reference to embodiments described below together with the accompanying drawings. The present disclosure may, however, be embodied in many different forms and should not be construed as being limited to the embodiments set forth herein, rather, these embodiments are provided such that the present disclosure will be thorough and complete, and will fully convey the concept of the present disclosure to those of ordinary skill in the art, and the present disclosure will be defined only by the concept of the claims.

Unless otherwise defined, all terms used herein, including technical and scientific terms, have the same meaning as commonly understood by one of ordinary skill in the art to which the present disclosure pertains. Terms such as those defined in commonly used dictionaries should be interpreted as having meanings consistent with their meanings in the context of the relevant art and will not be interpreted in an idealized or overly formal sense unless expressly so defined herein.

Shapes, sizes, ratios, angles, numbers, and the like in the drawings for describing the embodiments of the present disclosure are illustrative, and thus, the present disclosure is not limited to the illustrated details. In addition, in describing the present disclosure, detailed descriptions of related well-known technologies will be omitted if it is determined that they may unnecessarily obscure the subject matter of the present disclosure. When terms such as "comprises," "has," "includes," or "consists of" are used in the present specification, other parts may be added unless a limiting term such as "only" is used. Unless explicitly stated otherwise, the singular expressions include the plural expressions.

In interpreting components, they are construed as including a margin of error even if there is no separate explicit description.

When a positional relationship between two parts is described using terms such as "on," "above," "below," or "beside," one or more intervening parts may be present between the two parts unless a term such as "directly" or "immediately" is used.

When an element or layer is referred to as being 'on' another element or layer, it encompasses both the case where it is directly on the other element or layer and the case where other layers or elements are interposed therebetween. Like reference numerals denote like elements throughout the specification.

The dimensions and thicknesses of the components shown in the drawings are for convenience of description and do not necessarily limit the scope of the present disclosure.

The respective features of various embodiments of the present disclosure may be combined or coupled with each other, in whole or in part. As would be clearly understood by those skilled in the art, various technical interworking and operations are possible, and the embodiments may be implemented independently of each other or may be implemented together in an associated relationship.

An excrement collector 1 or 100 according to the present disclosure is to be attached to the buttocks of a user to collect excrement of the patient, and may include an excrement receptacle 30 or 130 configured to receive the excrement, a mounting unit 110 configured to surround the waist of the user or to be adhered to the skin of the user, and a temperature measurement unit 140 configured to measure the temperature of the excrement received in the excrement receptacle 30 or 130.

An excrement collection system having a temperature monitoring function according to an embodiment of the present disclosure may include the excrement collector 1 or 100, and may further include a first terminal device 200, a second terminal device 300, a user terminal device 400, a local repeater 500, a caregiver terminal device 800, a service server 700, and the like.

In an embodiment, the excrement collector 1 or 100 may be in a substantially bag-like shape and configured to be attached to the buttocks of a patient with limited mobility to collect excrement of the patient. The excrement collector 1 or 100 according to another embodiment of the present disclosure may be in the form of a diaper configured to be worn by an infant or an elderly person. That is, the excrement collector 1 or 100 according to the present disclosure may encompass any form that is attached to a user's buttocks and includes the excrement receptacle 30 or 130 configured to be attached to the buttocks of a user to receive excrement, the mounting unit 110 configured to surround the waist of the user or to be adhered to the skin of the user, and the temperature measurement unit 140 configured to measure the temperature of a perianal region of the user.

Referring to FIGS. 1, 2, and 3, the excrement collector 1 according to an embodiment of the present disclosure may be an apparatus configured to be attached to the buttocks of a patient to collect excrement of the patient.

The excrement collector 1 may include a main sheet part 10, an adhesive sheet part 20, and an excrement receptacle 30. In the corresponding embodiment, the mounting unit of the excrement collector 1 corresponds to the main sheet part 10 and the adhesive sheet part 20.

The main sheet part 10 may include a buttocks-shaped portion 11 formed in a shape corresponding to the buttocks of a patient, and a through-portion 13 formed through the center of the buttocks-shaped portion 11. The buttocks-shaped portion 11 may be formed in a shape corresponding to the buttocks of a patient. The through-portion 13, which is formed through the center of the buttocks-shaped portion 11 and has a substantially circular shape, may provide a path for excrement of the patient to be moved to the excrement receptacle 30.

The adhesive sheet part 20 may be formed on one surface of the main sheet part 10 in a shape corresponding to the main sheet part 10 such that the main sheet part 10 is adhered to the buttocks of the patient.

Here, the term "adhesion" is used to mean tackiness, which is distinguished from "bonding." "Bonding" refers to a state in which an adherend surface is joined through an interfacial bonding force generated by an adhesive, the interfacial bonding force depending on chemical interaction and mechanical interlocking between surface molecules of the bonded materials. "Adhesion," in contrast, refers to a degree of tackiness or stickiness with which objects cling to each other, and denotes a weaker attachment than "bonding."

The main sheet part 10 may further include slits 15 formed by being cut from a periphery toward the through-portion 13. The slits 15 may provide a space for the main sheet part 10 to be deformed according to the shape of the buttocks of the patient, thereby expanding the adhesion area of the main sheet part 10 to the buttocks of the patient.

The excrement receptacle 30 may be bonded to another surface of the main sheet part 10, specifically to a periphery defining the through-portion 13, to collect the excrement of the patient discharged through the through-portion 13.

The excrement receptacle 30 may be formed of a flexible, transparent or opaque, and sealable material, such as biodegradable vinyl, and may have a hollow cylindrical shape, wherein a base area of the cylindrical shape may be greater than or equal to the area of the through-portion 13.

The excrement collector 1 according to an embodiment of the present disclosure is an apparatus for allowing a bedridden patient to relieve themselves in situ. Specifically, with the through-portion 13 being aligned with the patient's anus, the adhesive sheet part 20 is adhered to the patient's buttocks to restrict displacement of the main sheet part 10 and the excrement receptacle 30 during defecation. Once the excrement is collected in the excrement receptacle 30, the adhesive sheet part 20 adhered to the patient's buttocks may be detached to dispose of the excrement.

The excrement receptacle 30 of the excrement collector 1 according to an embodiment of the present disclosure may extend from a periphery of the main sheet part 10 defining the through-portion 13 in a direction perpendicular to the direction in which the through-portion 13 is formed.

Specifically, the excrement receptacle 30 may be formed in the direction of penetration of the through-portion 13 and extend in a direction parallel to a plane defined by the main sheet part 10. This configuration is to allow the excrement receptacle 30 to be bent while being placed between both legs of the patient, when the main sheet part 10 is attached to the buttocks of the patient lying in a supine position.

A bonding part 33 is provided at one end of the excrement receptacle 30 such that the excrement receptacle 30 may be bonded to one surface of the main sheet part 10.

A space-forming part 31 is in close contact with another surface of the main sheet part 10 while being folded in a bellows-like shape to prevent air inflow into the collection space, and may be unfolded as the collection space expands when the patient's excrement is introduced through the through-portion 13.

If the excrement receptacle 30 is unfolded before the patient relieves themselves, air may flow into the unfolded collection space, which may lead to damage to the excrement receptacle 30 or cause it to detach from the patient's buttocks due to the influx of the patient's excrement into the sealed collection space.

Because the excrement collector 1 according to an embodiment of the present disclosure is in close contact with the other surface of the main sheet part 10 while being folded in a bellows-like shape to prevent air inflow into the collection space and then expands and unfolds as the patient's excrement is introduced through the through-portion 13, it is possible to prevent the inflow of air and avoid damage to the excrement receptacle 30 or detachment of the excrement receptacle 30 from the patient's buttocks, which would otherwise be caused by the influx of waste into a sealed collection space.

Referring to FIGS. 4 to 8, an excrement collector 100 according to an embodiment of the present disclosure may include the mounting unit 110, the excrement receptacle 30, and the temperature measurement unit 140.

The mounting unit 110 may include a through-portion 113, a concave portion 111, a convex portion 112, and a connection portion 115.

The concave portion 111 may be formed concavely in an arcuate shape from a periphery defining an outer surface of the mounting unit 110 toward the through-portion 113.

The concave portion 111 is disposed to face the patient's genitalia when the mounting unit 110 is attached to the patient's body, such that an adhesive sheet part 20 does not contact the patient's genitalia.

The convex portion 112 may be formed along the periphery while being connected to one side of the concave portion 111, and may be formed convexly in a direction away from the through-portion 113.

The mounting unit 110 may be formed in a shape in which one side and another side are symmetrical with respect to a virtual line connecting a central part of the concave portion 111 and a central part of the through-portion 113.

The connection portion 115 is formed along the periphery and may connect a convex portion 112 provided at one side of the through-portion 113 to another convex portion 112 provided at a position symmetrical to the convex portion 112, and may be positioned on an opposite side of the concave portion 111. In an embodiment, when the mounting unit 110 is folded along a connection line connecting a center of the concave portion 111 and a center of the connection portion 115, the mounting unit 110 may be overlapped to fit exactly. That is, it may be formed symmetrically with respect to the connection line.

The convex portion 112 may perform a function of expanding the adhesion area between the mounting unit 110 and the patient's buttocks.

The temperature measurement unit 140 may include a signal line 141, a thermistor 142, and a contact pad 143.

An upper surface of the mounting unit 110 is attached to a perianal region of the wearer, and an excrement receptacle 130 is coupled to a lower surface of the mounting unit 110. In an embodiment, the temperature measurement unit 140 may be fixed to the mounting unit 110 while the signal line 141 is positioned between the lower surface of the mounting unit 110 and the excrement receptacle 130.

The signal line 141 may include a first part P1 and a second part P2. The thermistor 142 may be provided at one end of the first part P1, and the contact pad 143 may be provided at another end of the second part P2.

In an embodiment, the first terminal device 200 may provide a current to the signal line 141 through the contact pad 143, and the thermistor 142 may change the current as its resistance varies according to a change in temperature, and the first terminal device 200 may derive the temperature change of the thermistor 142 by using this change in current.

The first part P1 may be provided inside the through-portion 113, and a portion of the second part P2 may be coupled to the mounting unit 110. The contact pad 143 may be provided outside the mounting unit 110, and the first terminal device 200 illustrated in FIG. 9 or the like may be detachably coupled to the contact pad 143. A temperature sensing result of the thermistor 142 may be transmitted to the contact pad 143 along the signal line 141, and the first terminal device 200 electrically connected to the contact pad 143 may receive and process the temperature sensing result.

The first part P1 is positioned to pass through a vicinity of the center of the through-portion 113, a portion of the second part P2 is coupled to the mounting unit 110, and the contact pad 143 is positioned outside the mounting unit 110. In an embodiment, if a position where the concave portion 111 is provided is defined as a 12 o'clock position of the mounting unit 110, the contact pad 143 may be positioned at a 10 to 11 o'clock position or a 1 to 2 o'clock position. Accordingly, a connection line or the like between the contact pad 143 and the first terminal device 200 may be naturally positioned near an inguinal region of the wearer, thereby reducing discomfort to the wearer, while a connection between the first terminal device 200 and the contact pad 143 may be stably maintained even when there is movement of the wearer.

A portion of the first part P1 and the thermistor 142 may be inserted into the anus of the wearer. For this purpose, it is preferable that a length obtained by subtracting the radius of the through-portion 113 from the length of the first part P1 is 10 mm to 20 mm. If the length of the first part P1 is too short, it is difficult for the portion of the first part P1 and the thermistor 142 to be stably inserted into the anus of the wearer, and if the length of the first part P1 is too long, it may cause discomfort to the wearer. In an embodiment, a portion of the temperature measurement unit 140 to be inserted into the anus may be formed of a material harmless to a human body, such as silicone, or may be provided with a surface coating layer.

FIGS. 5 and 6 illustrate a vertical coupling relationship among the mounting unit 110, the temperature measurement unit 140, and the excrement receptacle 130. The mounting unit 110 may include the main sheet part 10, and a film layer F1 may be coupled to a lower surface of the main sheet part 10. In an embodiment, the main sheet part 10 may be formed of a non-woven fabric material, and the adhesive sheet part 20 may be provided on the main sheet part 10. For example, an adhesive part 21 may be coupled to an upper surface of the main sheet part 10, and a release film part 23 may be coupled to an upper surface of the adhesive part 21. When the mounting unit 110 is attached to the wearer, the release film part 23 may be removed to allow the adhesive part 21 to be exposed.

In an embodiment, an upper portion of the excrement receptacle 130 may be coupled to the lower surface of the mounting unit 110, and the temperature measurement unit 140 may be coupled between the mounting unit 110 and the excrement receptacle 130.

FIG. 7 schematically illustrates the temperature measurement unit module 140-1 for mass-producing the temperature measurement unit 140, and FIG. 8 schematically illustrates an auxiliary film 146 of the temperature measurement unit 140. In an embodiment, the temperature measurement unit 140 may further include a cladding layer 144 in addition to the signal line 141, the thermistor 142, and the contact pad 143. The cladding layer 144 may be provided to surround an exterior of the signal line 141 and may serve to ensure insulation of the signal line 141. In addition, the cladding layer 144 may be formed of a flexible material to be easily bent or curved. Accordingly, even if the mounting unit 110 is bent according to a wearer's anatomy when attached around the wearer's anus, the temperature measurement unit 140 may be brought into close contact with the wearer's body along with the mounting unit 110. Furthermore, by bending the first part P1, a portion of the first part P1 and the thermistor 142 may be easily inserted into the wearer's anus.

In an embodiment, a manufacturing process may proceed in a state where a plurality of temperature measurement units 140 are fixed by a molded cladding 145. In the present disclosure, the structure in which the plurality of temperature measurement units 140 are fixed by the molded cladding 145 is referred to as the temperature measurement unit module 140-1. In an embodiment, the temperature measurement unit module 140-1 may be produced by supplying a material for the cladding layer 144 into a mold while the thermistor 142, the signal line 141, and the contact pad 143 are positioned in the mold, molding them, and then cutting them along a cutting line CL.

In an embodiment, the auxiliary film 146 may be further provided on an exterior of the cladding layer 144. The auxiliary film 146 may be implemented with the same material as the aforementioned film layer F1, and as a result, the temperature measurement unit 140 may be stably fixed to the film layer F1. The auxiliary film 146 may also be provided on a portion of the cladding layer 144 while in the form of the temperature measurement unit module 140-1. In an embodiment, the auxiliary film 146 may be coupled to the cladding layer 144 by using a thermal compression or thermal fusion method. While the auxiliary film 146 may be provided on only one of an upper surface and a lower surface of the cladding layer 144, it is advantageous for enhancing bonding strength to provide the auxiliary film 146 on both the upper and lower surfaces of the cladding layer 144 such that a first auxiliary film layer 146-1 on the upper surface and a second auxiliary film layer 146-2 on the lower surface are thermally fused while in contact with each other.

In an embodiment, an upper surface of the excrement receptacle 130 is in contact with a lower surface of the film layer F1 and a lower surface of the temperature measurement unit 140. That is, the upper surface of the excrement receptacle 130 may be fixed to the mounting unit 110 while in contact with the film layer F1, the auxiliary film 146, and the cladding layer 144.

In an embodiment, the film layer F1 and the auxiliary film 146 may be formed of a synthetic resin material, and preferably, a polyethylene (PE) material. In addition, a thickness of the film layer F1 and the auxiliary film 146 may be in a range of 0.02 mm to 0.10 mm, and more preferably, in a range of 0.03 mm to 0.05 mm, which is advantageous for enhancing bonding strength and ensuring the flexibility of the mounting unit 110. Furthermore, the excrement receptacle 130 may also be formed of the same material as the film layer F1 and the auxiliary film 146, in which case the bonding strength between the excrement receptacle 130 and the mounting unit 110 may be enhanced. A thickness of a film forming the excrement receptacle 130 may be in a range of 0.03 mm to 0.10 mm, and more preferably, in a range of 0.05 mm to 0.07 mm, thereby reducing the risk of damage to the excrement receptacle 130 while enhancing the bonding strength between the excrement receptacle 130 and the mounting unit 110. Meanwhile, the cladding layer 144 is an element for ensuring insulation of the signal line 141, but the cladding layer 144 itself has a limitation in bonding strength when bonded to a PE-based film, and as a result, there is a risk that the position of the thermistor 142 may vary. In an embodiment of the present disclosure, by providing the auxiliary film 146, the cladding layer 144 may be stably and firmly fixed between the mounting unit 110 and the excrement receptacle 130. In an embodiment, the bonding therebetween may be performed by using a thermal fusion method without a separate adhesive, thereby improving manufacturing efficiency. In FIG. 8, a width W1 of the cladding layer 144 may be implemented to be about 3 mm, and a width W2 of the auxiliary film 146 may be implemented to be about 6 mm. In addition, the cladding layer 144 may be implemented such that its width at the side of the thermistor 142 is narrower than its width at the side of the contact pad 143, whereby a process of inserting the thermistor 142 and the first part P1 into the wearer's anus may proceed seamlessly, and the wearer's discomfort may be reduced.

In an embodiment, as illustrated in FIG. 11, the temperature measurement unit 140 may further include an additional thermistor 142-1 provided at a position other than the thermistor 142 provided at one end of the first part P1 described above. For example, in a state where the mounting unit 110 is attached to a perianal region of a wearer ("User"), the additional thermistor 142-1 may be provided in a vicinity where the wearer's urine is discharged; accordingly, it is possible to monitor whether urine is discharged in real-time.

+Referring to FIG. 9, an excrement collection system having a temperature monitoring function according to an embodiment of the present disclosure may include the excrement collector 100, the first terminal device 200, and the second terminal device 300.

Because the excrement collector 100 is as described above, a redundant description thereof will be omitted.

The first terminal device 200 may be removably provided on the contact pad 143 of the temperature measurement unit 140, and may perform a function of generating temperature-related information by processing a current of the signal line 141 received through the contact pad 143. In addition, regarding a temperature monitoring result or the like, when a predetermined situation occurs, the first terminal device 200 may output whether the situation has occurred by using a method such as visual, auditory, or tactile methods. For example, the first terminal device 200 may be set to output a warning sound, flash a warning light, or vibrate when an abnormal situation occurs. Furthermore, the first terminal device 200 may perform a function of transmitting temperature-related information to another external device. In an embodiment, the first terminal device 200 may include a circuit board, a connection line, and a connection terminal. The circuit board may include a communication module, which may be implemented in a wired communication manner or a wireless communication manner. In addition, the connection line may provide a wired connection between the connection terminal device and the circuit board. Because the connection line is positioned around a patient's body, it is preferable that the connection line be formed of a flexible material. The connection terminal device may be attached to or detached from the contact pad 143.

In an embodiment, the first terminal device 200 may be connected to one temperature measurement unit 140. In this case, the information transmitted by the first terminal device 200 may include at least one of identification information and status information of the first terminal device 200, urine detection status, feces detection status, and measured temperature-related information.

The first terminal device 200 and the excrement collector 100 may be connected to each other by coupling the connection terminal device to the contact pad 143 in a state where the excrement collector 100 is attached to a perianal region of the wearer.

The second terminal device 300 may be connected to the first terminal device 200 through a wired or wireless communication network, and may provide a monitoring situation to management personnel such as nursing staff by analyzing information transmitted from the first terminal device 200. A communication scheme between the first terminal device 200 and the second terminal device 300 may be implemented in various ways, such as wired communication, LoRa wireless communication, RS-232 (communication between a microprocessor unit (MPU) and a wireless module), Wi-Fi, Bluetooth, Zigbee, or cellular. In an embodiment, an output device 310 connected to the second terminal device 300 to output a monitoring situation may be further provided. In an embodiment, the first terminal device 200 may transmit, to the second terminal device 300, a signal received through the contact pad 143. For example, when a situation in which an instantaneous temperature changes by 1°C or more occurs, the first terminal device 200 may immediately transmit situation occurrence information to the second terminal device 300. In this case, the transmission of information may be performed according to a predetermined cycle, or may be performed in a manner of immediate transmission when a specific situation occurs. In addition, the second terminal device 300 may send an information transmission request to the first terminal device 200 at a predetermined cycle (e.g., several times per 1 to 30 minutes).

In an embodiment, the temperature-related information generated by the first terminal device 200 may be information regarding a numerical value of current, and the second terminal device 300 may receive and process the information regarding the numerical value of current to derive an absolute temperature value, a relative temperature value, or a temperature change rate per hour, and may determine whether an abnormal situation (e.g., occurrence of a feces excretion event) has occurred.

In an embodiment, the temperature-related information generated by the first terminal device 200 may include an absolute or relative temperature value of current, a temperature change rate per hour, and whether an abnormal situation has occurred, and the second terminal device 300 may perform a function of storing and displaying the temperature-related information received from the first terminal device 200. In this case, even without the second terminal device 300, the first terminal device 200 alone may output visual, auditory, and tactile warnings regarding the abnormal situation.

Hereinafter, an application example of the excrement collection system having a temperature monitoring function according to an embodiment of the present disclosure will be described.

First, in a nursing hospital with a plurality of hospital rooms where a plurality of patients requiring care reside, the excrement collector 100 is worn by a patient with limited mobility and connected to the first terminal device 200. When implemented in this manner, approximately 1 to 20 first terminals 200 may be installed per hospital room, and one second terminal device 300 may be installed per 2 to 50 hospital rooms.

In an embodiment, one first terminal device 200 may be provided per wearer, or one per several wearers positioned adjacent to each other, to monitor temperature changes around a perianal region of the wearer and transmit this information to the second terminal device 300. In this case, the transmitted information may include a patient number, a current temperature of the thermistor 142, and status values of the first terminal device 200, such as a remaining battery level.

Through the second terminal device 300 or the output device 310 connected to the second terminal device 300, a manager may monitor the statuses of a plurality of wearers and immediately identify who the wearer with an abnormal issue is and in which hospital room they are located.

In an embodiment, when a temperature change is detected through the temperature measurement unit 140 and the temperature change is notified to the manager via the second terminal device 300, the manager in charge of the wearer with the abnormal issue may approach the wearer to check the excrement collector 100, and if actual defecation activity is confirmed, the manager may separate the contact pad 143 from the connection terminal, detach the excrement collector 100 from the wearer for disposal, and attach a new excrement collector 100 to the wearer. Subsequently, by coupling the contact pad 143 of the new excrement collector 100 to the connection terminal, the replacement of the excrement collector 100 may be completed.

Referring to FIG. 10, an excrement collection system having a temperature monitoring function according to another embodiment of the present disclosure may further include the user terminal device 400, the local repeater 500, the caregiver terminal device 800, a communication network 600, the service server 700, and the like.

The first terminal device 200 may provide temperature monitoring information to the user terminal device 400 (e.g., a wearer's smartphone), and the user terminal device 400 may transmit the temperature monitoring information to the service server 700 via the communication network 600 such as Wi-Fi or cellular.

The local repeater 500 may relay information transmission and reception among the first terminal device 200, the user terminal device 400, the second terminal device 300, and the server.

The service server 700 may perform a function of collecting, processing, and storing information related to monitoring of the wearer's temperature.

The service server 700 may transmit information related to monitoring of the wearer's temperature to the caregiver terminal device 800.

The caregiver terminal device 800 may access the service server 700 to request and receive the information related to monitoring of the wearer's temperature.

According to the present embodiment, the excrement collection system having a temperature monitoring function according to an embodiment of the present disclosure may be utilized even when the wearer lives at home rather than in a collective facility and a manager, such as a care worker, is in charge of a predetermined area. For example, when the first terminal device 200, which has collected information about monitoring of the wearer's temperature, registers the information through the wearer's user terminal device 400, the user terminal device 400 may transfer the information to the service server 700. Then, the service server 700 may transmit the monitoring information to the caregiver terminal device 800 of the manager in charge of the wearer. The manager may check which wearer has an abnormal situation and approach the wearer to perform necessary measures.

Although representative embodiments of the present disclosure have been described in detail, those of ordinary skill in the art to which the present disclosure pertains will understand that various modifications can be made to the above-described embodiments without departing from the scope of the present disclosure. Therefore, the scope of the present disclosure should not be limited to the described embodiments, but should be defined by the following claims as well as the equivalents to these claims.

### List of Reference Numerals for Major Elements

1, 100 : Excrement collector
10 : Main sheet part
11 : Buttocks-shaped portion
13, 113 : Through-portion
15 : Slit
20 : Adhesive sheet part
21 : Adhesive part
23 : Release film part
30, 130 : Excrement receptacle
31 : Space-forming part
33 : Bonding part
110 : Mounting unit
111 : Concave portion

### Industrial Applicability

The present disclosure may be applied to sanitary products for supporting hygienic excretion of patients, the elderly, or the like. Therefore, the present disclosure may be widely utilized for hygiene management and work efficiency improvement in various fields including home care, nursing facilities, and hospitals, as well as the medical and nursing industries. In particular, the present disclosure provides a structure that is in close contact with a wearer's body and may stably accommodate excrement so as not to leak to the outside, thereby significantly improving convenience for both the wearer and a manager. In addition, because it may be configured to be disposable or reusable, various industrial applications are possible in terms of production and distribution. Accordingly, the present disclosure has high industrial applicability across the entire auxiliary hygiene device industry.

## Claims

1. An excrement collection system having a temperature monitoring function, the excrement collection system comprising:
a mounting unit having one surface configured to adhere to a user's skin and a through-portion provided at an inner side thereof;
an excrement receptacle coupled to the other surface of the mounting unit and configured to accommodate excrement; and
a temperature measurement unit configured to measure a temperature of the excrement,
wherein the temperature measurement unit includes:
a signal line including a first part and a second part that extends from the other end of the first part;
a thermistor provided at one end of the first part; and
a contact pad provided at the other end of the second part,
wherein the first part is positioned inside the through-portion, and
at least a portion of the second part is coupled to the mounting unit.

2. The excrement collection system of claim 1, wherein a length obtained by subtracting a radius of the through-portion from a length of the first part is 10 mm to 20 mm.

3. The excrement collection system of claim 1, wherein the mounting unit includes a main sheet part having a film layer coupled to another surface thereof,
the temperature measurement unit includes a cladding layer provided outside the signal line to ensure insulation, and an auxiliary film covering at least a portion of the cladding layer, and
one surface of the excrement receptacle is coupled to the mounting unit while in contact with the film layer, the cladding layer, and the auxiliary film.

4. The excrement collection system of claim 3, wherein the excrement receptacle, the film layer, and the auxiliary film are formed of the same material.

5. The excrement collection system of claim 1, wherein the mounting unit further includes a concave portion, and
if a position where the concave portion is provided is defined as a 12 o'clock position on the mounting unit, the contact pad is positioned at a 10 to 11 o'clock position or a 1 to 2 o'clock position on the mounting unit.

6. The excrement collection system of claim 5, wherein the mounting unit further includes a convex portion and a connection portion, the convex portion being positioned at 3 o'clock and 9 o'clock positions on the mounting unit, and the connection portion being positioned at a 6 o'clock position on the mounting unit.

7. The excrement collection system of claim 1, further comprising:
a first terminal device removably provided on the contact pad and configured to generate temperature-related information by processing a current of the signal line; and
a second terminal device connected to the first terminal device via a wired or wireless communication network to collect the temperature-related information.
